# EUROPÄISCHE PATENTSCHRIFT

(11) **EP 2 388 310 B1**
(45) Veröffentlichungstag und Bekanntmachung des Hinweises auf die Patenterteilung: **14.03.2018**
(21) Anmeldenummer: 11004050.8
(22) Anmeldetag: 17.05.2011
(51) Int. Cl.: C12M 1/00

(54) **PHOTOBIOREAKTOR**
PHOTOBIOREACTOR
PHOTOBIORÉACTEUR

(30) Priorität: 21.05.2010 DE 102010021154
(43) Veröffentlichungstag der Anmeldung: 23.11.2011
(73) Patentinhaber: Karlsruher Institut für Technologie (KIT), 76131 Karlsruhe (DE)
(72) Erfinder: Posten, Clemens, 73137 Karlsruhe (DE); Jacobi, Anna, 76187 Karlsruhe (DE); Steinweg, Christian, 76185 Karlsruhe (DE); Lehr, Florian, 67069 Ludwigshafen (DE); Rosello, Rosa, 60486 Frankfurt am Main (DE)
(74) Vertreter: Gärtner, Stephan

(56) Entgegenhaltungen:
- EP-A1- 0 749 773
- WO-A1-2009/059111
- DE-C1- 4 411 486
- US-A1- 2003 170 884
- US-A1- 2008 293 132

## Beschreibung

Die vorliegende Erfindung betrifft eine Vorrichtung und ein Verfahren zur Vermehrung phototropher Mikroorganismen, im Folgenden wird die Vorrichtung auch als Photobiorektor bezeichnet.

Bioreaktoren oder Fermenter sind Vorrichtungen, um Mikroorganismen unter möglichst idealen Bedingungen zu kultivieren, damit eine optimale Ausbeute an Zellen oder von Zellen produzierten Substanzen erreicht wird. Bei herkömmlichen Bioreaktoren ist vor Allem der Eintrag von Nährstoffen für die Organismen, die Temperatur und ggf. Belüftung für die Ausbeute ausschlaggebend.

Bei der Kultur phototropher Organismen in einem Photobioreaktor kommt ein weiterer entscheidender Wachstumsfaktor hinzu, nämlich der zusätzliche Eintrag von Licht, den phototrophe Mikroorganismen als Energiequelle benötigen, um sich zu entwickeln.

Die Produktion von Mikroalgen ist derzeit noch auf wenige tausend Tonnen beschränkt, Das Interesse an Algenbiomasse ist groß, da Mikroalgen ein vielversprechender Grundstoff für die Produktion von Energieträgern wie Biodiesel, Biomethan oder Wasserstoff sind. Weiterhin kann Algenbiomasse einer stofflichen Nutzung zugeführt werden, beispielsweise durch medizinisch aktive Inhaltsstoffe, oder als Nahrungs- bzw. Futtermittel.

Diebekannten Verfahren zur Gewinnung von Algenbiomasse, nutzen entweder Verfahren in offenen Systemen oder in geschlossenen Photobioreaktoren. Die geschlossenen Photobioreaktoren bieten den Vorteil, die Reaktionsparameter gezielt zu steuern, Kontaminationen zu minimieren und deutlich höhere Produktivitäten zu erzielen.

Für die Produktion von Biomasse aus phototrophen Mikroorganismen sind als Energiequelle Licht, als Kohlenstoffquelle CO₂ oder andere organische Moleküle sowie geeignete Nährstoffe in wässriger Lösung erforderlich. Die verwendeten Mikroorganismen werden zunächst unter sterilen Bedingungen angezüchtet und vermehrt. Danach wird das so genannte Inokulat zusammen mit dem Nährmedium in den Photobioreaktor eingebracht. Zur bestmöglichen Vermehrung und Gewinnung von Biomasse werden eine Begasung, die optimale Temperatur und der pH-Wert eingestellt.

Der Gaseintrag erfolgt dabei häufig von oben über die Oberfläche der Kultivierungsflüssigkeit, die der Luft ausgesetzt ist. Aufgrund der geringen Fläche und der geringen CO₂-Konzentration der Luft, ist der Gaseintrag hierbei jedoch gering. Alternativ können Gasgemische mit angereichten CO₂-Gehalten von unten durch Blasen eingetragen werden. Dadurch erhöhen sich Austauschfläche und CO₂-Konzentrationsgradient. Hierfür sind allerdings entsprechende Pumpenergien nötig, die sich negativ in der Energiebilanz auswirken.

Im Stand der Technik werden verschiedene geschlossene Photobioreaktoren beschrieben.

DE 199 16 597 A1 beschreibt einen sogenannten Airlift-Photobioreaktor. Dieser Typ von Röhrenreaktoren ist dadurch gekennzeichnet, das er durch die von unten kommende vertikale Begasung eine gute Durchmischung erzielt und zusätzlich durch eine Vergrößerung der Oberfläche mit Hilfe von Fortsätzen und Einbauten und einen sog. Flashing-Light-Effekt induziert werden kann. Beim Flashing-Light Effekt handelt es sich um einen Effekt, bei dem durch schnelle Wechsel der Beleuchtungsintensitäten (Hell-Dunkel-Zyklen) erhöhte Wachstumsraten erzielt werden können. Diese Zyklen entstehen dadurch, dass die Algen aufgrund der Geometrie des Reaktors und der Begasung eine turbulente Strömung erfahren, wodurch sie schnell zwischen gut beleuchteten Stellen und beschatteten Stellen fluktuieren. Der Eintrag von CO₂ in Photobioreaktoren über Membranen wird in US 2009/0305389 A1 beschrieben. Hierbei handelte es sich im Unterschied zur vorliegenden Erfindung um einen Folienreaktor. Die Membranen sind nicht in die Oberfläche des Reaktors integriert, sondern als steife Hohlkörpermembranen in der Suspension realisiert.

In DE 10 2008 311 769 A1 wird eine Flachbauweise vorgeschlagen, wobei die Wachstumskammern jeweils voneinander getrennt sind und über Zuleitungs- bzw. Ableitungskammern mit Wasser und CO₂ versorgt werden. Es ist also kein Durchflussreaktor. Vorzugsweise erfolgt die Durchmischung durch walzenförmige Rotation der Kulturflüssigkeit durch Gasblasen. Der Aufbau dieser Photobioreaktoren ist derart, dass die einzelnen Module seriell oder parallel angeordnet werden können. Der Eintrag von Lichtenergie ist lediglich dadurch gewährleistet, dass die verwendeten Materialien vorzugsweise lichtdurchlässig sind und optional aus wellenlängenschiebendem Material gefertigt sind. Eine konstruktionsbedingte Oberflächenvergrößerung für die Lichteinbrechung ist nicht erwähnt. WO 2008059111 A1 offenbart einen Photoreaktor für die Algenkultur mit transparenten Wandungen. Ein Grundproblem der Kultivierung phototropher Mikroorganismen ist die geringe Toleranz der meisten Spezies für hohe Lichtstärken. Für die meisten Mikroalgen treten Sättigungserscheinungen wesentlich unterhalb der maximalen Tageslichtintensität von ca. 200 Watt/m² auf. Andererseits soll die maximal mögliche Lichtmenge ausgenutzt werden, um hohe Photonenkonversionseffizienzen zu erreichen. Bei den bisher veröffentlichten Bioreaktoren findet keine Vergleichmäßigung und Verdünnung des eindringenden Lichtfeldes statt. Die Intensitäten, mit denen die Mikroorganismen beleuchtet werden, weisen im Einzelnen nicht vermessene, undefinierte und hohe Gradienten auf. Daher können diese Reaktoren keine volle Produktivitäten entfalten.

Sowohl bei offenen als auch bei geschlossenen Systemen wird die Algenbiomasse meist durch geeignete Vorrichtungen (Schaufelräder, Pumpen, Luftströme im Reaktor, Reaktordesign) bewegt, um ein Absetzen der Algenbiomasse zu verhindern und um eine verbesserte Belichtung der Algen zu realisieren. Hierdurch wird ein erheblicher Energieeintrag in die Systeme erforderlich, der die Energiebilanz schmälert.

Weiterhin werden in der Regel aufwändige Gerüste eingesetzt, die die Reaktoren in der Senkrechten z.B. gegen Winddruck stabilisieren. Alternativ wird auch ein Gewächshaus genutzt, das jedoch bereits selbst höhere Kosten verursacht.

Um wirtschaftlich auch im Hinblick auf die spätere Aufarbeitung zu arbeiten, ist neben der flächenbezogenen Produktivität insbesondere die Konzentration an Mikroalgen im Medium entscheidend. Bislang werden geschlossene Reaktoren mit Konzentrationen von nicht mehr als 2 g/l betrieben auch um Selbstbeschattung der Kultur bei hohen Zelldichten zu vermeiden.

Trotz der neuartigen technologischen Ansätze gelingt es beim aktuellen Stand der Technik nicht, den Preis für Photobioreaktoren in den Bereich von 25 €/m² zu senken, für den Studien eine wirtschaftliche energetische Nutzung der Mikroalgen sehen. Des Weiteren ist der Aufwand an Hilfsenergie für Begasung und Mischung bei weitem zu hoch. Typische Werte, soweit überhaupt bekannt, liegen über 5 W/m², verbrauchen also bereits den zu erwartenden stofflichen Energiegewinn aus den Mikroalgen. Bislang veröffentliche Reaktorsysteme sind also für den Einsatz im energetischen Bereich nicht geeignet. Für die Wasserstoffgewinnung ergeben sich weitere technische Probleme.

Um die Nachteile des Stands der Technik überwinden, ist es Aufgabe der Erfindung, einen Bioreaktor für die Kultivierung von phototrophen Mikroorganismen sowie ein Verfahren zum Betreiben desselben vorzuschlagen. Weiterhin ist es Aufgabe der Erfindung eine Verwendung des Bioreaktors für die Produktion von Energieträgern vorzuschlagen. Der Bioreaktor soll insbesondere eine optimierte Lichteinbrechung gewährleisten, sodass eine Kultur mit hoher Zelldichte möglich ist. Der Eintrag von Pumpenergien für die Umwälzung, Begasung und Durchmischung soll möglichst gering gehalten werden. Aufgrund seines Aufbaus kann der Bioreaktor kostengünstig hergestellt und betrieben werden, wodurch eine wirtschaftlich sinnvolle Produktion an Biomasse bzw. Energieträgern gegeben ist.

Die vorliegende Erfindung löst die Aufgabe durch den in Anspruch 1 beanspruchten Bioreaktor, das in Anspruch 8 beanspruchte Verfahren und die in Anspruch 11 beanspruchte Verwendung. Bevorzugte Ausführungsformen des Bioreaktors und des Verfahrens sind in den rückbezogenen Ansprüchen beschrieben. Anspruch 7 bezieht sich auf eine Anordnung mehrerer Bioreaktoren nach den Ansprüchen 1 bis 6. Es handelt sich um einen Bioreaktor für die Kultivierung von phototrophen Mikroorganismen. Der Aufbau des Bioreaktors umfasst zwei Platten, eine obere lichtdurchlässige Platte und eine untere Platte. Beide Platten sind im Wesentlichen parallel übereinander angeordnet, sodass zwischen den Platten ein durchgehendes Kultivierungsvolumen bereitgestellt ist. Der Abstand zwischen den beiden Platten, welcher der Höhe des Kultivierungsvolumens entspricht, ist gering, vorzugsweise von 0,1 mm bis 40 mm, besonders bevorzugt von 1 mm bis 10 mm. Durch die geringe Schichtdicke ist die Wahrscheinlichkeit für die gegenseitige Beschattung der Organismen niedriger als bei größeren Schichtdicken.

Damit der Abstand zwischen den beiden Platten über die gesamte Fläche des Bioreaktors konstant ist, können optional Abstandshalter zwischen der oberen und der unteren Platte eingebaut werden, die verhindern, dass die obere Platte aufgrund Ihrer Beschaffenheit durchhängt, wodurch der Abstand zwischen den beiden Platten geringer würde. Dieses "Durchhängen" ist abhängig von der Plattengröße, deren Schichtdicke und vor allem von den verwendeten Materialien. In Abhängigkeit dieser Faktoren sollte die Anzahl und die Verteilung der Abstandshalter angepasst werden.

Die Form beider Platten ist durch eine Mehrzahl von Erhebungen und Vertiefungen gekennzeichnet, welche in der oberen und der unteren Platte vorhanden sind, sodass das Kultivierungsvolumen über die Fläche die gleiche Schichtdicke hat. Die Erhebungen und Vertiefungen sind parallel zueinander und erstrecken sich über die gesamte Reaktorbreite in einer sich regelmäßig wiederholenden Geometrie. Diese Geometrie gibt dem Bioreaktor bzw. dem Kultivierungsvolumen vorzugsweise eine Wellen- oder Zickzackform. Im Gegensatz zu Flachplattenreaktoren aus dem Stand der Technik, die eine weitgehend ebene Kultivierungsoberfläche haben, erlaubt diese Wellen- oder Zickzackform eine Verdünnung des Lichteintrags. Insbesondere beim Betrieb der Bioreaktoren im Sonnenlicht, dessen Lichtintensität weit über der Sättigungsintensität der phototrophen Organismen liegt, kann eine größere Kulturfläche beleuchtet werden, bei einer gleichzeitig moderateren Lichtintensität.

Der Bioreaktor umfasst mindestens einen Zu- und Ablauf, durch welche zentrale Funktionen bedient werden können. Zum einen kann der Bioreaktor mit einer Kultur beschickt werden, zum anderen kann die Kultur durch entsprechende Fördermechanismen im Kultivierungsvolumen umgewälzt werden und letztlich kann die Kultur auch wieder abgelassen bzw. abgeerntet werden.

In einer bevorzugten Ausführung sind die Zu- und Abläufe derart angeordnet, dass die Strömungsrichtung der Kultur innerhalb des Bioreaktors parallel entlang der Erhebungen oder Vertiefungen verläuft. Bei dieser Strömungsrichtung ergibt sich der Vorteil, dass der Strömungswiderstand geringer ist, als wenn Flüssigkeitsstrom über die Erhebungen und Vertiefungen verläuft. Dadurch kann der Bioreaktor mit einer geringeren Pumpenergie betrieben werden. Ein zu hoher Pumpendruck kann konstruktionsbedingt zu einer Deformierung des Bioreaktors führen, was zu vermeiden ist.

Über die Zu- und Abläufe können mehrere dieser Bioreaktoren miteinander verbunden werden, sodass ein größeres Kultivierungsvolumen entsteht. Hierbei ist eine serielle oder parallele Verknüpfung der Bioreaktoren möglich.

In einer bevorzugten Ausführung beträgt die Gesamthöhe der Erhebungen und Vertiefungen von 0,5 cm bis 30 cm, besonders bevorzugt von 2 cm bis 10 cm.

In einer weiteren bevorzugten Ausführung des erfindungsgemäßen Bioreaktors beträgt die Grundfläche desselben von 0,5 m² bis 50 m², besonders bevorzugt von 1 m² bis 25 m². Die Grundfläche des Bioreaktors ist hierbei nicht gleichzusetzen mit der Oberfläche des Kultivierungsvolumens, welches aufgrund der Erhebungen und Vertiefungen entsprechend größer ist.

Das Verhältnis der Grundfläche des Bioreaktors zur Oberfläche des Kultivierungsvolumens liegt vorzugsweise im Bereich von 1:2 bis 1:10.

In einer bevorzugten Ausführung des erfindungsgemäßen Reaktors beträgt die Anzahl an Erhebungen und Vertiefungen pro Meter Reaktorbreite von 10 bis 100.

Die lichtdurchlässige obere Platte des Bioreaktors ist bevorzugter Weise mit einer IR-reflektierenden Beschichtung ausgestattet. Verglichen mit Licht im sichtbaren Wellenlängenbereich, das für die Photosynthese der phototropen Organismen genutzt wird, ist das Infrarotlicht in erster Linie als Wärmestrahlung zu betrachten, die den Reaktor beim Betrieb im Sonnenlicht zu bestimmten Tageszeiten stark aufheizen kann. Durch die IR-reflektierende Beschichtung kann ein Großteil der Wärmestrahlung entfernt werden.

Um die Beleuchtung des Kultivierungsvolumens zu optimieren, kann in einer bevorzugten Ausführung des Bioreaktors das durch das Kultivierungsvolumen transmittierte Licht mittels einer lichtreflektierenden Beschichtung an der unteren Platte zurück in das Kultivierungsvolumen reflektiert werden. Dadurch wird auch ein Großteil des transmittierten Lichts den phototrophen Organismen zur Verfügung, die von der Reaktorunterseite her über die Lichtreflexion beleuchtet werden. Die Verluststrahlung wird mittels der lichtreflektierenden Beschichtung minimiert.

Ein weiterer entscheidender Punkt für die Effizienz eines Photobioreaktors ist die optimale Versorgung mit CO₂. Die Begasung findet über permeable Membranen statt, die in der unteren Platte integriert sind. Ein Vorteil ist, dass der Energieeintrag für die Begasung durch den Einsatz von Begasungsmembranen, bei denen der Phasenübergang gasförmiggelöst bereits im Membranmaterial stattfindet, minimiert wird. Die Form der Begasungsmembranen ist variabel. Beispielsweise kann die Membran schlauchförmig sein und das Kulturmedium durchziehen (z.B. entlang der Vertiefungen), wobei die Membran an der unteren Platte angebracht sind. Alternativ kann es eine Flachmembran sein, welche die untere Platte teilweise permeabel macht für den Durchtritt von CO₂. In diesem Fall ist es vorteilhaft den Bioreaktor durch eine zusätzliche untere Abdeckung an der Unterseite hermetisch zu verschließen. Dadurch ist es möglich, dass ein Gasstrom, der ggf. mit CO₂ angereichert wurde, zwischen der unteren Abdeckung und der unteren Platte durch geleitet wird, wobei das CO₂ durch die Membran in die Kultur eindiffundieren kann. Die Begasung über Membranen ist vorteilhaft, um den Einsatz von hydraulischer und pneumatischer Hilfsenergie zu minimieren. Die Gasbildung wird zum Stofftransport genutzt, so dass die notwendige Pumpenergie minimiert wird. Im Idealfall arbeitet die Versorgung mit CO₂ über einen höheren CO₂-Partialdruck, der durch Anreicherung der Gasversorgung mit CO₂ erreicht wird.

Des Weiteren kann in einer weiteren Ausführung des erfindungsgemäßen Bioreaktors die untere Platte mit Sensoren ausgestattet bzw. die Sensoren in die untere Platte integriert sein, welche zur Erfassung verschiedener Kultivierungsparameter dienen. Zu den Kultivierungsparametern gehören beispielsweise die Gelöstkonzentrationen von O₂ und CO₂, der pH-Wert, die optische Dichtre und vor Allem die Temperatur. Die Erfassung von Kultivierungsparametern dient zur Steuerung des Bioreaktors, sodass optimale Kulturbedingungen bereitgestellt werden können. Insbesondere kann der sensorgesteuerte Bioreaktor autark arbeiten, was einen kostengünstigen dezentralen Betrieb ermöglicht.

Optional wird der erfindungsgemäße Bioreaktor mit einer oberen Abdeckung ausgestattet. Diese kann zum Schutz vor extremen Witterungseinflüssen, wie z.B. Hagelschlag o.Ä. dienen.

Weiterhin kann diese obere Abdeckung als eine hermetische Abdichtung des Bioreaktors dienen. Wird der Bioreaktor beispielsweise zur Wasserstoffproduktion mit Hilfe von Mikroalgen betrieben, kann das entstandene Wasserstoffgas durch das Material der oberen Platte hindurch diffundieren. In diesem Fall darf die obere Platte nicht aus Glas oder einem gleichfalls Wasserstoff-unpermeablen Material gefertigt sein, hingegen muss die obere Abdeckung aus Glas oder einem anderen Wasserstoff-unpermeablen Material gefertigt sein, sodass das Gas zwischen der oberen Abdeckung und der oberen Platte aufgefangen wird. Wasserstoff kann aufgrund seiner Größe und Eigenschaften durch die meisten Polymermaterialien hindurch diffundieren.

Die Erfindung betrifft auch ein Verfahren zum Betreiben eines Bioreaktors umfassend folgende Verfahrensschritte:
a) Bereitstellen eines erfindungsgemäßen Bioreaktors wie in den vorigen Abschnitten beschrieben;
b) Das Kultivierungsvolumen des Bioreaktors wird mit einer Flüssigkultur befüllt, die phototrophe Mikroorganismen enthält;
c) Die Mikroorganismen werden mit Nährstoffen versorgt, die im Wesentlichen durch Diffusion und Konvektion im Kulturmedium verteilt werden;
d) Die Kultur wird bei Tageslicht inkubiert;
e) Die Mikroorganismen werden abgeerntet, wenn die Zellen als Biomasse verwendet werden oder in den Zellen befindliche Stoffe extrahiert werden sollen, oder die ins Medium eindiffundierten Stoffwechselprodukte, wie z.B. Wasserstoff, werden abgetrennt.

Die Kultur wird als statische Kultur (Batch-Kultur) oder als kontinuierliche Kultur betrieben.

Vorzugsweise wird der Bioreaktor in horizontaler Ausrichtung, d.h. im Wesentlichen parallel zur Erdoberfläche betrieben.

Unter einer Batch-Kultur versteht man ein diskontinuierliches Kulturverfahren, bei dem der Bioreaktor einmal mit einer Kultur beschickt wird und bis zur Abernte darin verbleibt. Ein Umwälzen der Kultur bzw. die Zufuhr von essentiellen Stoffen wie CO₂ ist auch hier erforderlich.

Eine kontinuierliche Kultur funktioniert im Permanentbetrieb. Wachstum, Vermehrung und Ernte der Kultur bzw. der Stoffwechselprodukte erfolgt kontinuierlich. Das bedeutet auch, dass die phototrophen Mikroorganismen durchgehend mit Nährstoffen versorgt werden.

In einer bevorzugten Ausführung des Verfahrens wird die die Kultur in Schritt c) über gaspermeablen Membranen mit Kohlendioxid versorgt. Die Versorgung mit Nährstoffen kann im erfindungsgemäßen Verfahren aufgrund der geringen Schichtdicke des Bioreaktors im Wesentlichen durch Diffusion und durch leichte Konvektion (Temperaturkonvektion und leichtes Umwälzen des Kulturmediums) ausreichend gewährleistet werden. Zu hohe Pumpenergien sollten vermieden werden, da der Reaktor konstruktionsbedingt aufblähen kann.

Sofern das erfindungsgemäße Verfahren für die Produktion gasförmiger Stoffwechselprodukte angewendet wird, werden die entstandenen Gase in Verfahrensschritt e) vorzugsweise mittels geeigneter Membranen abgetrennt werden. Hierzu kann beispielsweise ein Teil der Reaktorfläche durch Membranmaterialien ersetzt werden. Durch diesen Aufbau wäre eine Abtrennung der Gase über die obere oder untere Platte möglich. Hierzu ist allerdings eine weitere Abdeckung oberhalb und unterhalb der beiden Reaktorplatten erforderlich, da sonst die Gase entweichen.

Bei der Produktion von gasförmigen Stoffwechselprodukten und gleichzeitigem Beschicken des Reaktors mit CO₂ können Gasgemische entstehen. Dies kann aber durch eine dem Fachmann geläufige CO₂-Abtrennung gelöst werden.

Der hier vorgestellte Bioreaktor sowie das Verfahren zu dessen Betrieb eignen sich insbesondere dafür, dass phototrope Mikroorganismen autark auf kostengünstige Weise produziert werden können. Die geschlossene Bauweise eines solchen Bioreaktors erlaubt beispielsweise einen Betrieb auf dezentralen Flächen mit aridem, sonnereichen Klima. Die erfindungsgemäßen Bioreaktoren können zur Produktion von Wasserstoff mit Hilfe geeigneter phototropher Mikroorganismen verwendet werden.

Die Erfindung wird im Folgenden mit Ausführungsbeispielen und folgenden Figuren erläutert.
**Fig. 1** Schematische Darstellung eines erfindungsgemäßen Bioreaktors.
**Fig. 2** Schematisch Querschnittszeichnung durch den erfindungsgemäßen Bioreaktor.

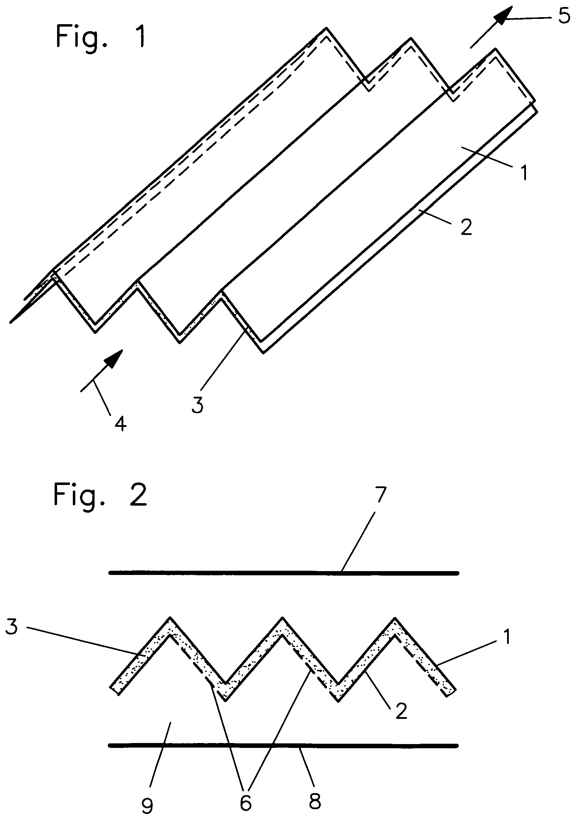

In **Fig. 1** ist schematisch der Aufbau des Bioreaktors in zickzackförmiger Bauweise dargestellt. Das Kultivierungsvolumen **3** befindet sich zwischen oberer Platte **1** und unterer Platte **2.** In diesem Kultivierungsvolumen **3** befindet sich die Kultur mit phototrophen Mikroorganismen. Die Lichteinbrechung erfolgt von oben. Es ist zu erkennen, dass die Oberfläche der Kultur größer ist als die Gesamtfläche, die der Reaktor einnimmt. Aufgrund der Zickzackstruktur trifft die Lichtstrahlung immer in einem Winkel auf die Oberfläche der Kultur, wodurch die Strahlungsintensität verringert und die insgesamt bestrahlte Fläche vergrößert wird.

Der Bioreaktor in **Fig. 1** ist weiterhin mit einem Zulauf **4** und einem Ablauf **5** ausgestattet. Die bevorzugte Strömungsrichtung im Kultivierungsvolumen **3** ist parallel zu den Erhebungen und Vertiefungen.

**Fig. 2** zeigt einen Querschnitt durch den Bioreaktor aus **Fig. 1****,** der noch zusätzlich mit einer oberen Abdeckplatte **7** und einer untere Abdeckplatte **8** ausgestattet ist. In dieser Darstellung ist zu erkennen, wie die CO₂-Zufuhr aus dem unteren Gasraum **9** durch eine Membran **6** in das Kultivierungsvolumen **3** gelangt. Diese Art des CO₂-Eintrags ist im Wesentlichen auf Diffusion basiert und bedarf daher keines hohen Überdrucks.

### Bezugszeichenliste

- 1: Obere Platte
- 2: Untere Platte
- 3: Kultivierungsvolumen
- 4: Zulauf
- 5: Ablauf
- 6: Membran
- 7: Obere Abdeckplatte
- 8: Untere Abdeckplatte
- 9: Gasraum für CO₂ Zufuhr

## Patentansprüche

1. Bioreaktor für die Kultivierung von phototrophen Mikroorganismen, umfassend ein durchgehendes Kultivierungsvolumen mit einem Zu- und einem Ablauf, das zwischen zwei übereinander angeordneten Platten, einer oberen lichtdurchlässigen Platte und einer unteren Platte, mit einem Abstand bereitgestellt ist, wobei in die untere Platte gaspermeable Membranen, Membranstreifen und/oder Membranschläuche integriert sind, **dadurch gekennzeichnet, dass** beide Platten parallel zueinander jeweils eine Mehrzahl von Verformungen mit Erhebungen und Vertiefungen in regelmäßig wiederholender Geometrie aufweisen, wobei der Abstand zwischen den beiden Platten über die gesamte Fläche des Bioreaktors konstant ist.

2. Bioreaktor nach Anspruch 1, wobei die Mehrzahl von Verformungen mit Erhebungen und Vertiefungen eine Wellen- oder Zickzackform hat.

3. Bioreaktor nach Anspruch 1 bis 2, wobei die Gesamthöhe der Erhebungen und Vertiefungen von 1 cm bis 20 cm beträgt.

4. Bioreaktor nach Anspruch 1 bis 3, wobei der Abstand zwischen der oberen und der unteren Platte von 0,1 mm bis 40 mm beträgt.

5. Bioreaktor nach Anspruch 1 bis 4, wobei die lichtdurchlässige obere Platte mit IR-reflektierender Beschichtung ausgestattet ist.

6. Bioreaktor nach Anspruch 1 bis 5, wobei die untere Platte eine lichtreflektierende Beschichtung aufweist.

7. Anordnung mehrerer Bioreaktoren nach Anspruch 1 bis 6, wobei die Bioreaktoren über die Zu- und Abläufe seriell oder parallel miteinander verbunden sind.

8. Verfahren zum Betreiben eines Bioreaktors umfassend folgende Verfahrensschritte:
a) Bereitstellen eines Bioreaktors nach Anspruch 1 bis 6,
b) Bereitstellen einer Flüssigkultur mit phototrophen Mikroorganismen, die in dem Kultivierungsvolumen des Bioreaktors befindlich ist,
c) Versorgung der Mikroorganismen mit Nährstoffen durch Diffusion und Konvektion der Stoffe im Kulturmedium,
d) Inkubieren der Kultur im Tageslicht,
e) Abernten der Mikroorganismen oder der ins Medium eindiffundierten Stoffwechselprodukte, wobei die Kultur als statische Kultur (Batch-Kultur) oder als kontinuierliche Kultur betrieben wird.

9. Verfahren nach Anspruch 8, wobei die Kultur in Schritt c) über gaspermeable Membranen mit Kohlendioxid versorgt wird.

10. Verfahren nach Anspruch 8 oder 9, wobei gasförmige Stoffwechselprodukte in Verfahrensschritt e) über die Flüssigkeitsoberfläche transportiert werden und mittels Membranen oder gaspermeablen Reaktormaterialien abgetrennt werden.

11. Verwendung von Bioreaktoren nach einem der Ansprüche 1 bis 6 zur Produktion von Wasserstoff mittels phototropher Mikroorganismen.

## Claims

1. Bioreactor for the cultivation of phototropic micro-organisms, comprising a continuous cultivation volume with an inlet and an outlet, which is provided between two plates arranged above one another, an upper light-permeable plate and a lower plate, with a spacing interval, wherein gas-permeable membranes, membrane strips, and/or membrane tubes are integrated into the lower plate, **characterised in that** both plates, parallel to one another, comprise in each case a plurality of shapings with elevations and depressions in a regularly repeating geometrical pattern, wherein the spacing interval between the two plates is constant over the entire surface of the bioreactor.

2. Bioreactor according to claim 1, wherein the plurality of shapings with elevations and depressions have a wave form or zigzag form.

3. Bioreactor according to claim 1 to 2, wherein the overall height of the elevations and depressions amounts to 1 cm to 20 cm.

4. Bioreactor according to any one of claims 1 to 3, wherein the spacing interval between the upper and lower plates amounts to 0.1 mm to 40 mm.

5. Bioreactor according to any one of claims 1 to 4, wherein the light-permeable upper plate is provided with an IR-reflecting coating.

6. Bioreactor according to any one of claims 1 to 5, wherein the lower plate exhibits a light-reflecting coating.

7. Arrangement of a plurality of bioreactors according to any one of claims 1 to 6, wherein the bioreactors are connected by the inlets and outlets in series or in parallel with one another.

8. Method for operating a bioreactor, comprising the following method steps:
a) provision of a bioreactor in accordance with any one of claims 1 to 6,
b) provision of a liquid culture with phototrophic micro-organisms, which is located in the cultivation volume of the bioreactor,
c) supply of the micro-organisms with nutrients by diffusion and convection of the substances in the culture medium,
d) incubation of the culture in daylight,
e) harvesting of the micro-organisms or of the metabolic products diffused into the medium, wherein the culture is treated as a static culture (batch culture) or as continuous culture.

9. Method according to claim 8, wherein the culture in step c) is supplied with carbon dioxide via gas-permeable membranes.

10. Method according to claim 8 or 9, wherein gaseous metabolic products in method step e) are transported over the liquid surface and are separated by means of membranes or gas-permeable reactor materials.

11. Use of bioreactors in accordance with any one of claims 1 to 6, for the production of hydrogen by means of phototropic micro-organisms.

## Revendications

1. Bioréacteur destiné à la culture de microorganismes phototrophes comprenant un volume de culture continu ayant une entrée et une sortie, et qui est situé entre deux plaques superposées et situées à distance l'une de l'autre, une plaque supérieure perméable à la lumière et une plaque inférieure, dans laquelle sont intégrées des membranes, des bandes membranaires et/ou des tubes membranaires perméables au gaz,
**caractérisé en ce que**
les deux plaques comportent chacune, un ensemble de déformations parallèles ayant des bossages et des cavités réparties selon une géométrie régulière répétitive, la distance entre les deux plaques étant constante sur la totalité de la surface du bioréacteur.

2. Bioréacteur conforme à la revendication 1,
dans lequel les déformations de l'ensemble de déformations ayant des bossages et des cavités ont une forme ondulée ou en zigzag.

3. Bioréacteur conforme à la revendication 1 ou 2,
dans lequel la hauteur globale des bossages et des cavités est comprise entre 1 cm et 20 cm.

4. Bioréacteur conforme à l'une des revendications 1 à 3,
dans lequel la distance entre la plaque supérieure et la plaque inférieure est comprise entre 0,1 mm et 40 mm.

5. Bioréacteur conforme à l'une des revendications 1 à 4,
dans lequel la plaque supérieure perméable à la lumière est équipée d'un revêtement réfléchissant les IR.

6. Bioréacteur conforme à l'une des revendications 1 à 5,
dans lequel la plaque inférieure comporte un revêtement réfléchissant la lumière.

7. Agencement de plusieurs bioréacteurs conforme à l'une des revendications 1 à 6,
dans lequel les bioréacteurs sont reliés en série ou en parallèle par les entrées et les sorties.

8. Procédé de gestion d'un bioréacteur comprenant les étapes suivantes consistant à :
a) se procurer un bioréacteur conforme à l'une des revendications 1 à 6,
b) se procurer une culture liquide renfermant des microorganismes phototrophes située dans le volume de culture du bioréacteur,
c) alimenter les microarganismes avec des nutriments par diffusion et convection de ces nutriments dans le milieu de culture,
d) faire incuber la culture à la lumière du jour,
e) récolter les microorganismes ou les produits d'échange de matière ayant diffusé dans le milieu, la culture étant gérée sous la forme d'une culture statique (culture par fournées) ou sous la forme d'une culture continue.

9. Procédé conforme à la revendication 8,
selon lequel dans l'étape c) la culture est alimentée en dioxyde de carbone par l'intermédiaire de membranes perméables aux gaz.

10. Procédé conforme à la revendication 8 ou 9,
selon lequel des produits d'échange de matières gazeux sont transportés dans l'étape e) sur la surface du liquide et sont séparés au moyen de membranes ou de matériaux réacteurs perméables aux gaz.

11. Utilisation de bioréacteurs conforme à l'une des revendications 1 à 6, pour la production d'hydrogène au moyen de microorganismes phototrophes.
